# EUROPEAN PATENT APPLICATION

(11) **EP 0 710 486 A1**
(43) Date of publication of application: **08.05.1996**
(21) Application number: 94919890.7
(22) Date of filing: 12.07.1994
(51) Int. Cl.: A61K 45/00, A61K 31/18

(54) **REMEDY FOR URINATION DISORDER ACCOMPANYING PROSTATIC HYPERTROPHY**

(30) Priority: 14.07.1993 JP 197999/93
(71) Applicant: YAMANOUCHI PHARMACEUTICAL CO. LTD., Tokyo 103 (JP); YAMANOUCHI U.K. LIMITED, West Byfleet, Surrey KT4 6RA (GB)
(72) Inventor: ASANO, Masaharu, Itabashi-ku, Tokyo 174 (JP); TAKENAKA, Toichi, Abiko-shi, Chiba 270-11 (JP); MICHEL, Martin C., Biochemisches Forschungslabor, D-45122 Essen (DE)
(74) Representative: Geering, Keith Edwin
(86) International application number: JP9401135
(87) International publication number: WO9502419

(57) **Abstract**

A remedy for urination disorder accompanying prostatic hypertrophy that contains an α_{1c} receptor subtype selective blocker as the active ingredient and does not adversely affect the blood pressure.

## Description

### TECHNICAL FIELD

This invention relates to an agent useful in treating urinary outlet obstruction associated with benign prostatic hyperplasia, without exerting influence upon blood pressure, which contains an α_{1c} receptor subtype-selective blocking agent as the active ingredient.

### BACKGROUND ART

It is said that one in five males of fifty-five years or more has certain prostatic hypertrophy symptoms and, in view of the increase in the aging population of the society, the need for treating urinary outlet obstruction associated with benign prostatic hyperplasia has been increasing year by year.

Though treatment of urinary outlet obstruction associated with benign prostatic hyperplasia is carried out mainly by surgical therapy rather than drug therapy in the current medical care, therapeutic effects of 5α reductase inhibitors and α₁ receptor blocking agents have recently been drawing attention of the related fields and several of such drugs have approved or in application for their indication.

Originally, α₁ receptor blocking agents were noticed as hypotensive drugs, and synthesis of their various derivatives has been studied up to the present. On the other hand, it has been found in recent years that certain α₁ receptor blocking agents developed as hypotensive drugs (for example, prazosin, terazosin and the like) also have therapeutic effects on urinary outlet obstruction associated with benign prostatic hyperplasia, and such an indication was added as the second medical use. Though efficacy of these drugs can be recognized by clinical tests, it has been pointed out that their hypotensive action as the original medical utility rather becomes a risk of causing side effects when used in said area of treatment, so that clinicians hesitate in administering them to old patients.

In consequence, there is a demand in medical aspect for development of a drug useful in treating urinary outlet obstruction associated with benign prostatic hyperplasia without exerting influence upon blood pressure.

Based on the cloning of the receptor gene, the α₁ receptor has been re-classified from the conventional subtypes (α_{1N}, α_{1H}, α_{1L}) into different four subtypes (α_{1A}, α_{1B}, α_{1C}, α_{1D}), and it has been reported recently that 75% of the α₁ receptor in human prostata tissue is the α_{1C} receptor subtype (D.T. Price *et al*., *J*. *Urology*, 149(4), 324A, 1993) and that the α_{1C} receptor subtype is mainly concerned in the contraction of human prostata caused by α blocking agents (D.J. Smith *et al*., *J*. *Urology*, 149(4), 434A, 1993). It has also been reported that the α_{1B} receptor subtype is mainly concerned in blood pressure (E. Suzuki *et al*., *Mol*. *Pharmacol*., 38, 725 - 736, 1990). In consequence, it is suggested that a compound capable of acting selectively upon the α_{1C} receptor subtype will become an ideal drug for use in the treatment of urinary outlet obstruction associated with benign prostatic hyperplasia, which does not exert influence upon blood pressure. However, actual reports on such a drug have not been made and virtually nothing is known even about a compound highly selective for the α_{1C} receptor subtype.

As described above, creation of an excellent drug for use in the treatment of urinary outlet obstruction associated with benign prostatic hyperplasia is even now a medically important subject.

The present inventors have synthesized sulfamoyl-substituted phenetylamine derivatives having α₁ receptor blocking action and found that R(-)-5-[2-[[2-(o-ethoxyphenoxy)ethyl]amino]propyl]-2-methoxybenzenesulfonamide (general name, tamusulosin; to be referred to as "compound A" hereinafter) shows excellent effects (Japanese Patent Publication (*kokoku*) No. 62-52742).

### DISCLOSURE OF THE INVENTION

The inventors of the present invention have further continued clinical studies on the compound A as a therapeutic agent for urinary outlet obstruction associated with benign prostatic hyperplasia and unexpectedly found that, unlike the case of other α₁ receptor blocking agents, the compound A and its enantiomer S(+)-5-[2-[[2-(o-ethoxyphenoxy)ethyl]amino]propyl]-2-methoxybenzenesulfonamide (to be referred to as "compound B" hereinafter) are α_{1C} receptor subtype-selective blocking agent and useful for the treatment of urinary outlet obstruction associated with benign prostatic hyperplasia without exerting influence upon blood pressure (in other words, it was found that an α_{1C} receptor subtype-selective blocking agent becomes a therapeutic agent for urinary outlet obstruction associated with benign prostatic hyperplasia, which does not exert influence upon blood pressure), hence resulting in the accomplishment of the present invention.

Accordingly, the present invention relates to an agent for the treatment of urinary outlet obstruction associated with benign prostatic hyperplasia, without exerting influence upon blood pressure, which contains an α_{1C} receptor subtype-selective blocking agent as an active ingredient.

More particularly, the present invention provides a therapeutic agent for urinary outlet obstruction associated with benign prostatic hyperplasia showing no influence on blood pressure.

Both of the terms "does not exert influence upon blood pressure" and "showing no influence on blood pressure" mean that "it does not exert substantial influence upon blood pressure".

The present invention is based on the finding of the excellent medicinal utility of a compound which shows highly selective blocking effect on the α_{1C} receptor subtype, and examples of compounds which show the α_{1C} receptor subtype-selective blocking action include, though not particularly limited, the sulfamoyl-substituted phenetylamine derivatives and salts thereof having α₁ receptor blocking action disclosed in Japanese Patent Publication (*kokoku*) No. 62-52742.

A preferred compound among them is R(-)-5-[2-[[2-(o-ethoxyphenoxy)ethyl]amino]propyl]-2-methoxybenzenesulfonamide or a salt thereof.

More preferred compound is S(+)-5-[2-[[2-(o-ethoxyphenoxy)ethyl]amino]propyl]-2-methoxybenzenesulfonamide or a salt thereof.

The aforementioned compound which is the active ingredient of the pharmaceutical preparation of the present invention is used in the treatment as a free salt or a nontoxic acid addition salt with an organic or inorganic acid.

Illustrative examples of such salts include acid addition salts with mineral acids such as hydrochloric acid, sulfuric acid, nitric acid, phosphoric acid, hydrobromic acid, hydroiodic acid and the like or with organic acids such as acetic acid, oxalic acid, succinic acid, citric acid, maleic acid, malic acid, fumaric acid, tartaric acid, picric acid, methanesulfonic acid, ethanesulfonic acid and the like and salts with acidic amino acids such as glutamic acid, aspartic acid and the like.

Also included in the pharmaceutical preparation of the present invention are hydrates, various solvates and polymorphic forms of the aforementioned compound.

### INDUSTRIAL APPLICABILITY

The present invention is characterized in that it includes an α_{1C} receptor subtype-selective blocking agent and is useful as a urinary outlet obstruction treating agent showing no influence on blood pressure.

The excellent α_{1C} receptor subtype-selective blocking action of the pharmaceutical preparation of the present invention was confirmed by the following method.
[Affinity of [³H] prazosin and compounds A and B for the cloned α₁ receptor subtype]

The present inventors have evaluated the affinity of [³H]prazosin and compounds A and B for the cloned α₁ receptor subtype by carrying out the receptor binding experiments of a labeled compound.

The following describes experimental methods and results together with discussions.

### [Methods]

### I. Transfection of receptor-encoding gene:

Expression vectors used in this experiment are pCMVα_{1A} which contains an *Eco*RI-*Pst*I 2520 bp fragment of rat α_{1A} receptor subtype cDNA, pcDV1Rα_{1B} which contains a 2573 bp fragment containing the entire coding region of α_{1B} receptor subtype cDNA (J.W. Lomasney *et al*., *J*. *Biol*. *Chem*., 266, 6365 - 6369, 1991), and pBCα_{1c} which contains the entire coding region of bovine α_{1C} receptor subtype and was constructed in accordance with a reported method (D.A. Schwinn *et al*., *J*. *Biol*. *Chem*., 265, 8183 - 8189, 1990). COS-7 cells were transfected with these three expression plasmid vectors using a DEAE-dextran method additionally involving chloroquine and dimethyl sulfoxide steps for transient expression (S. Suryanarayana and B. K. Kobilka, *Methods*, 3, 193 - 204, 1991). After 4 days of the transfection, these cells were again suspended in an ice-cold buffer for binding experiment (50 mmol/l Tris, 0.5 mmol/l EDTA, pH 7.5) and homogenized using Tissumeizer for 10 seconds at a maximum speed and then twice for 20 seconds at 2/3 speed. The thus obtained homogenate was centrifuged at 50,000 g for 20 minutes, and the resulting pellet was again suspended in the buffer for binding experiment to a concentration of 0.5 to 2 mg/ml.

### II. Receptor binding experiment of labeled compound:

In accordance with a reported method (M. C. Michel *et al*., *Naunyn-Schmiedeberg's Arch*. *Phamacol*., 347, 180 - 185, 1993), binding of [³H] prazosin to a membrane preparations obtained from the COS-7 cells transfected with each receptor subtype cDNA was carried out. A 100 µl portion of each membrane sample suspension was incubated at 25°C for 45 minutes together with the indicated concentration of [³H] prazosin. This incubation was terminated by quick filtration under a reduced pressure using Whatman GF/C filter paper. Non-specific binding was defined as binding in the presence of 10 µmol/l of phentolamine. A series of six concentrations of [³H] prazosin was used in the saturation binding experiment, and the concentration of [³H] prazosin in the competitive binding experiment was set to 200 pmol/l which is close to the Kb value of the compound. The protein content was determined in accordance with the Bradford method using bovine IgG as an standard. In the competitive binding experiment, each compound was evaluated by 3 to 4 tests using at least two separate batches of transfected cells.

### III. Evaluation of data:

The result was shown as the average value ± SEM of n times of tests.

### [Results]

[³H] Prazosin showed high affinity for the specific binding site of COS-7 cells transfected with the gene coding for rat α_{1B}-, bovine α_{1C}- or rat α_{1A/D}-receptor subtype and bound almost equally (Table 1), but specific binding of [³H] prazosin was not detected in COS-7 cells which were not transfected with the gene coding for α₁-receptor subtype (data not shown).

**Table 1**

| | α_{1B} | α_{1C} | α_{1A/D} |
|---|---|---|---|
| K_{d} (pM) | 178 ± 87 | 252 ± 51 | 148 ± 15 |
| B_{MAX} (fmol/mg protein) | 1673 ± 1203 | 492 ± 177 | 240 ± 87 |
| n | 5 | 6 | 8 |

Competitive binding experiments were carried out using COS-7 cells in which rat α_{1B}-, bovine α_{1C}- or rat α_{1A/D}-receptor subtype was expressed. All of these compounds showed competitive antagonism for [³H] prazosin binding, having a Hill coefficient of approximately 1. Table 2 shows negative logarism values (-log values) of Ki values and data on the α_{1C}/α_{1B} receptor subtype selectivity obtained by these experiments.

**Table 2**

| | α_{1B} | α_{1C} | α_{1A/D} | α_{1C}(Ki)/α_{1B}(Ki) |
|---|---|---|---|---|
| Compound A | 9.06 ± 0.16 | 10.64 ± 0.28 | 10.06 ± 0.11 | 38 |
| Compound B | 6.98 ± 0.23 | 9.46 ± 0.35 | 7.86 ± 0.05 | 302 |

### [Discussion]

Since [³H] prazosin as the labeled compound showed similar affinity for all of the cloned α₁-receptor subtypes, it was judged that [³H] prazosin has no selectivity for these α₁-receptor subtypes.

To the contrary, as is evident from Table 2, the compounds A and B showed markedly higher selectivity for the α_{1C}-receptor than the α_{1B}-receptor.

Based on the above test results, it was revealed that the compounds A and B are selective blocking agent for the α_{1C} receptor subtype.

On the other hand, it has already been confirmed by human clinical tests that the compound A is useful in treating urinary outlet obstruction associated with benign prostatic hyperplasia without exerting influence upon blood pressure and hardly shows side effects (K. Kawabe *et al*., *J*. *Urol*., 1990, 144, 908 - 912).

Thus, it was confirmed that the α_{1C} receptor subtype-selective blocking agent is useful in treating urinary outlet obstruction associated with benign prostatic hyperplasia without exerting influence upon blood pressure, by selectively acting upon the prostata tissue. It was confirmed also that compounds A and B have high α_{1C} receptor subtype selectivity and low toxicity and therefore are useful as α_{1C} receptor blocking agents in treating urinary outlet obstruction associated with benign prostatic hyperplasia without exerting influence upon blood pressure.

### (Formulation Example)

Pharmaceutical preparations which contain compounds of the present invention having the effect as α_{1C} receptor subtype-selective blocking agents or pharmaceutically acceptable salts thereof as an active ingredient (including admixture preparations with other drug components) may be administered preferably by making them into oral dosage forms such as tablets, capsules, powders, fine granules, granules, pills, solutions and the like using generally used pharmaceutical carriers, excipients, diluents and other additives, but they can also be made into parenteral dosage forms such as injections, suppositories, ointments, adhesive preparations and the like.

Examples of such additives include starches such as corn starch, potato starch and the like, crystalline cellulose, calcium sulfate, calcium lactate, synthetic aluminum silicate, calcium hydrogenphosphate, anhydrous silicic acid, magnesium aluminate metasilicate, carboxymethylcellulose calcium, magnesium stearate, talc, hydrogenated plant oil, fatty acid mono, di or triglyceride, hydroxypropylcellulose, polyvinyl pyrrolidone and other generally used carriers, excipients, lubricants, disintegrating agents, binders, emulsifying agents, wetting agents, antiseptic agents, dispersing agents, stabilizing agents, solubilizing or solubilization enhancing agents, correctives, smell correctives, and the like. The tablet (oral preparation) shown in the following is an example of the dosage form.

| Composition | 0.2 mg tablet |
|---|---|
| Compound A or B | 0.2 mg |
| Lactose | 78.2 |
| Corn Starch | 18.1 |
| Hydroxypropylcellulose | 8 |
| Magnesium Stearate | 0.5 |
| Total | 100 mg |

Compound A or B (1.2 g) was mixed with 489.2 g of lactose in a polyethylene bag. The mixture was pulverized using a sample mill. The thus pulverized mixture (470.4 g) was uniformly mixed with 108.8 g of corn starch in a fluidized granulation coating machine. To this was sprayed 180 g of 10% hydroxypropylcellulose solution to effect granulation. After drying, the resulting granules were passed through a 20 mesh screen, mixed with 3 g of magnesium stearate and then applied to a rotary tablet making machine to produce tablets, each weighing 100 mg, making use of a die/punch system of ⌀ 8.5 mm x 7.8 R.

Clinical dose of the drug of the present invention is optionally decided in response to its degree of blocking action for α_{1C} receptor subtype and its degree of selectivity for other receptor subtypes such as α_{1B}. That is, it is desirable that the dose is selected within such a range that it is sufficient enough to express the α_{1C} receptor subtype blocking action and it does not or hardly exert influence upon blood pressure changes caused by α_{1B} and other receptor subtypes, and the dose for each drug of the present invention is optionally selected by methods known to those skilled in the art.

Though it varies depending on the symptoms, body weight and age of each patient to be administered and on the administration method, the dose of, for example, compound A may be generally from 0.05 to 2.0 mg, preferably from 0.1 to 0.8 mg, per day per adult in the case of oral administration, or generally from 0.005 to 20 mg per day per adult in the case of parenteral administration, and the daily dose recited above may be administered once a day or by dividing it into 1 to several doses a day.

In the case of compound B, its dose may be generally from 0.5 to 80 mg, preferably from 1.0 to 30 mg, per day per adult in the case of oral administration, and generally from 0.05 to 800 mg per day per adult in the case of parenteral administration.

When the drug of the present invention is used in the practical treatment, its effective dose is administered to each vertebrate which requires the treatment by applying one of the aforementioned administration method. The term "vertebrate" as used herein is not particularly limited and includes human and other primates, mammals (dogs, cats, cattle, swine, horses and the like), fishes, birds and the like, of which human and other primates and mammals are preferred, most particularly human.

## Claims

1. A therapeutic agent of urinary outlet obstruction associated with benign prostatic hyperplasia, which does not exert influence upon blood pressure, said agent comprising an α_{1c} receptor subtype-selective blocking agent as an active ingredient.

2. The therapeutic agent according to claim 1, wherein said α_{1c} receptor subtype-selective blocking agent is S(+)-5-[2-[[2-(o-ethoxyphenoxy)ethyl]amino]propyl]-2-methoxybenzenesulfonamide or a salt thereof.

3. The therapeutic agent according to claim 1, wherein said α_{1c} receptor subtype-selective blocking agent is R(-)-5-[2-[[2-(o-ethoxyphenoxy)ethyl]amino]propyl]-2-methoxybenzenesulfonamide or a salt thereof.

4. A method for treating urinary outlet obstruction associated with benign prostatic hyperplasia in a vertebrate, which does not exert influence upon blood pressure, said method comprising administering an effective amount of an α_{1c} receptor subtype-selective blocking agent to said vertebrate.

5. The therapeutic method according to claim 4, wherein said α_{1c} receptor subtype-selective blocking agent is S(+)-5-[2-[[2-(o-ethoxyphenoxy)ethyl]amino]propyl]-2-methoxybenzenesulfonamide or a salt thereof.

6. The therapeutic method according to claim 4, wherein said α_{1c} receptor subtype-selective blocking agent is R(-)-5-[2-[[2-(o-ethoxyphenoxy)ethyl]amino]propyl]-2-methoxybenzenesulfonamide or a salt thereof.

7. Use of an α_{1c} receptor subtype-selective blocking agent for the manufacture of a medicament for treating urinary outlet obstruction associated with benign prostatic hyperplasia which does not exert influence upon blood pressure.

8. The use according to claim 7, wherein said α_{1c} receptor subtype-selective blocking agent is S(+)-5-[2-[[2-(o-ethoxyphenoxy)ethyl]amino]propyl]-2-methoxybenzenesulfonamide or a salt thereof.

9. The use according to claim 7, wherein said α_{1c} receptor subtype-selective blocking agent is R(-)-5-[2-[[2-(o-ethoxyphenoxy)ethyl]amino]propyl]-2-methoxybenzenesulfonamide or a salt thereof.

10. A method for generating selective α_{1c} receptor subtype-blocking action by administering to a vertebrate S(+)-5-[2-[[2-(o-ethoxyphenoxy)ethyl]amino]propyl]-2-methoxybenzenesulfonamide, R(-)-5-[2-[[2-(o-ethoxyphenoxy)ethyl]amino]propyl]-2-methoxybenzenesulfonamide, or a salt thereof.
